Europäisches Patentamt

European Patent Office

Office européen des brevets

(19)

(11) Veröffentlichungsnummer : **0 115 547**
**B1**

(12) **EUROPÄISCHE PATENTSCHRIFT**

(45) Veröffentlichungstag der Patentschrift :
03.12.86

(51) Int. Cl.⁴ : **C 12 Q  1/70**, G 01 N 33/48

(21) Anmeldenummer : 83100905.5

(22) Anmeldetag : 01.02.83

(54) Verfahren zur quantitativen Bestimmung der mutagenen Wirkung von chemischen Stoffen auf Phagen durch eine in vitro durchgeführte Untersuchung.

(43) Veröffentlichungstag der Anmeldung :
15.08.84 Patentblatt 84/33

(45) Bekanntmachung des Hinweises auf die Patenterteilung : 03.12.86 Patentblatt 86/49

(84) Benannte Vertragsstaaten :
AT CH DE FR GB LI SE

(56) Entgegenhaltungen :
DE-A- 3 018 900
MUTATION RESEARCH, Band 65, 1979, M. HOLLS-
TEIN et al. "Short-term tests for carcinogens and
mutagens", Seiten 133-226
MUTATION RESEARCH, Band 66, 1979, R. GLATT et
al. "Mutagenicity of 43 structurally related heterocyclic compounds and its relationship to their carcinogenicity", Seiten 307-328
STUDIA BIOPHYSICA, Band 33, Heft 2, 1972, G.
RONTO et al. "Ein kinetisches Modell zur Strahlenschädigung von viraler und bakterieller DNS", Seiten
121-130
Die Akte enthält technische Angaben, die nach dem
Eingang der Anmeldung eingereicht wurden und die
nicht in dieser Patentschrift enthalten sind.

(73) Patentinhaber : RADELKIS Elektrokémiai Müszergyárto Szövetkezet
Laborc u. 1
H-1300 Budapest (HU)

(72) Erfinder : Györgyi, Rontò, Dr.
Öv u. 150
H-1141 Budapest (HU)
Erfinder : István, Sugár, Dr.
Ervin u. 26
H-1026 Budapest (HU)
Erfinder : Szombathelyi, Agnes, Dr.
Hunyadi u. 5
H-1039 Budapest (HU)
Erfinder : Imre, Tarján, Dr.
Fehérvári u. 145
H-1119 Budapest (HU)

(74) Vertreter : Beszédes, Stephan G. Dr.
Münchener Strasse 80a Postfach 1168
D-8060 Dachau (DE)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

**Beschreibung**

Die Erfindung betrifft ein neues Verfahren zur quantitativen Bestimmung der mutagenen Wirkung von chemischen Stoffen auf Phagen durch eine *in vitro* durchgeführte Untersuchung.

Zur Klärung des schädlichen (in erster Linie mutagenen und karzinogenen beziehungsweise Krebs erzeugenden) Wirkung der chemischen Stoffe wäre nach dem gegenwärtigen Standpunkt das zweckmäßige Verfahren eine aus 3 Schritten bestehende Reihenuntersuchung (Bridges : Mutat. Res. *26* [1974], 335 bis 340), das sich auf 3 unterschiedliche Organisationsstufen bezieht. In der Praxis erfolgt die Untersuchung jedoch auf 1, eventuell 2, der 3 Stufen.

Die erste Stufe stellen die *in vitro* durchgeführten Reihenuntersuchungen, bei denen mehr oder minder zuverlässige annähernde Informationen über die mutagene Wirkung der untersuchten chemischen Stoffe erhalten werden, dar. Zur Klärung der karzinogenen (und teratogenen beziehungsweise Mißbildungen verursachenden) Wirkung der chemischen Stoffe ist diese Untersuchungsstufe nicht geeignet, da die verwendeten Untersuchungsorganismen (Mikroorganismen) von zu niederer Ordnung sind ; auf Grund der allgemeinen biologischen Kenntnisse ist es jedoch sehr wahrscheinlich, daß die unter Bedingungen *in vitro* eine mutagene Wirkung aufweisenden Stoffe in Organismen von höherer Ordnung karzinogen (oder teratogen) wirken werden. Daher werden die chemischen Stoffe, welche sind bei *in vitro* erfolgenden Untersuchungen als mutagen erwiesen, gewöhnlich nicht noch *in vivo* durchgeführten Reihenuntersuchungen unterworfen, sondern eindeutig als schädlich eingestuft.

Die Aufgabe der die zweite Stufe darstellenden *in vivo* durchgeführten Reihenuntersuchungen besteht darin, daß vom Gesichtspunkt der karzinogenen Wirkung die etwaige aktivierende Wirkung des tierischen Stoffwechsels auf die Chemikalien, welche sich bei *in vitro* durchgeführten Untersuchungen als inaktiv (unschädlich) erwiesen, untersucht wird. Obwohl es auch vorkommen kann, daß die Chemikalien, welche sich bei *in vitro* durchgeführten Untersuchungen als mutagen erwiesen, gerade durch die im tierischen Organismus ablaufenden Stoffwechselvorgänge (zum Beispiel Abbau) ihre schädliche Wirkung verlieren, werden diese Chemikalien wie bereits erwähnt im allgemeinen nicht mehr unter Bedingungen *in vivo* untersucht. Die *in vivo* erfolgenden Untersuchungen erfordern Organismen höherer Ordnung (für Laboratoriumszwecke geeignete Kleintiere) und gewöhnlich langwierige Versuche und sind mit wesentlich höherem Aufwand verbunden als die *in vitro* durchgeführten Untersuchungsverfahren.

Die dritte Stufe der Reihenuntersuchungen bilden die Humanuntersuchungen. Diese Untersuchungen erfolgen dann, wenn das Ausmaß des durch die Verwendung des chemischen Stoffes verursachten Risikos in Fällen, in welchen sich der chemische Stoff in den 2 vorherigen Reihenuntersuchungen als unschädlich oder aber in 1 der 2 Reihenuntersuchungen zwar als schädlich erwies, seine Verwendung jedoch unumgänglich ist, bestimmt werden soll. Diese Untersuchungen müssen gewöhnlich an menschlichen Gewebekulturen durchgeführt werden, so daß es klar ist, daß sie mit recht hohem Aufwand verbunden sind.

Wie aus dem Obigen hervorgeht, sind die *in vitro* durchgeführten Untersuchungen von grundlegender Bedeutung für die Bestimmung der Verwendbarkeit der chemischen Stoffe. Wenn diese Untersuchungen keine zuverlässigen Ergebnisse liefern oder aber zur entsprechenden Charakterisierung des untersuchten chemischen Stoffes nicht geeignet sind, kann es vorkommen, daß von der weiteren Untersuchung wertvoller Stoffe oder ihrer Verwendung Abstand genommen wird beziehungsweise die schädliche Wirkung, welche schon auch in den *in vitro* durchgeführten Untersuchungen hätte auftreten müssen, nur durch komplizierte und mit hohem Aufwand verbundene weitere Untersuchungen nachgewiesen werden kann. Neben der Zuverlässigkeit und ausreichenden Auswertbarkeit besteht eine weitere Anforderung an die *in vitro* durchgeführten Untersuchungen darin, daß sie einfach, schnell und mit geringem Aufwand durchführbar sein sollen ; heutzutage wäre es wünschenswert, daß sich diese Untersuchung nicht nur auf Medikamente, sondern auch auf die Untersuchung aller, beispielsweise in der Industrie, Landwirtschaft und in den Haushalten vorkommenden, chemischen Stoffe erstreckt.

Bei den bekannten *in vitro* durchgeführten Untersuchungsverfahren wird im allgemeinen die Mutation von Mikroorganismen (gewöhnlich Bakterien) untersucht. Von diesen Verfahren wird der sogenannte Ames-Test (Ames und Mitarbeiter : Proc. Natl. Acad. Sci., USA *70* [1973], 2 281) verhältnismäßig oft angewandt, wobei die auxotrophen Mutanten von Salmonella typhimurium mit dem zu untersuchenden chemischen Stoff behandelt werden und die vom letzteren hervorgerufene Reversion untersucht wird. Auch die von anderen Verfassern an anderen Objekten, zum Beispiel Mutanten von E. coli und S. cerevisiae, ausgearbeiteten Verfahren (Pueyo : Mutat. Res. *54* [1978], 311 ; Glatt und Mitarbeiter : Mutat. Res. *66* [1979], 307 ; Glatt und Mitarbeiter : Mutat. Res. *66* [1979], 329) beruhen auf einem ähnlichen Prinzip. Der gemeinsame Nachteil dieser Verfahren besteht darin, daß die Untersuchungen über die mutagene Wirkung des Stoffes meistens nur eine bejahende oder verneinende Antwort geben, die Wirkungsstärke aber quantitativ nicht charakterisieren, also nicht entsprechend ausgewertet werden können. Ein weiterer Nachteil ist, daß das Erhalten der entsprechenden auxotrophen Mutantenbakterienstämme eine mit verhältnismäßig hohem Aufwand verbundene und arbeitsintensive Aufgabe, welche auch viel Sachkenntnis erfordert, ist. Diese Verfahren zur Bestimmung der mutagenen Wirkung nutzen gewöhnlich nur das Endergebnis des Vorganges, mit der Kinetik, das heißt dem zeitlichen Ablauf, der mutagenen Wirkung des untersuchten chemischen Stoffes sind sie jedoch nicht befaßt.

Ferner ist aus der deutschen Offenlegungsschrift 30 18 900 ein Toxizitätstestverfahren für chemische Substanzen einschließlich der Untersuchung ihrer Eignung zur Induzierung von Mutationen und Begünstigung der Tumorbildung, bei welchem Nematoden verwendet werden, wobei als Kulturmedium für diese eine Phosphatpufferlösung verwendet werden kann, bekannt. Dieses Verfahren hat aber den Nachteil, daß die Nematoden eine Generationszeit von 2 bis 4 Tagen haben, also die Untersuchung und Auswertung der verschiedenen funktionellen und morphologischen Abnormitäten der ersten und zweiten Tochtergenerationen mindestens 4 Tage erfordert. Auch macht das Verfahren der deutschen Offenlegungsschrift 30 18 900 eine Statistik nur aus einer geringen Anzahl (je 20) von $F_1$- und $F_2$-Individuen. Ferner ermöglicht zwar das Verfahren de deutschen Offenlegungsschrift 30 18 900 das Charakterisieren der Abnormitäten, welche mit verschiedenen Konzentrationen der untersuchten Chemikalie hervorgerufen wurden, mit verschiedenen Prozentzahlen. Diese Prozentzahlen sind aber nicht nur für die Wirksamkeit der gegebenen Chemikalie, sondern auch für deren Konzentration charakteristisch, sie geben also keine Möglichkeit zum Vergleich der chemischen Stoffe vom Standpunkt der mutagenen Wirksamkeit (mutagenen Aktivität).

Die quantitative Bestimmung beziehungsweise Charakterisierung der unter Bedingungen *in vitro* auftretenden mutagenen Wirkungen befindet sich im Versuchsstadium. Von Glatt und Mitarbeitern (Mutat. Res. *66* [1979], 307) wurde ein willkürlicher Mutagenitätsindex, bei dessen Berechnung die an den von den 4 Salmonella-Mutanten (TA 1535, TA 1537, TA 98 und TA 100) für empfindlich befundenen 3 Stämmen (TA 100, TA 98, TA 1537) gemessene mutagene Wirkung, das heißt die durch 1 nMol chemischen Stoff hervorgerufene revertante Koloniezahlzunahme im Vergleich zur spontan revertierten Koloniezahl, mit unterschiedlichen Faktoren multipliziert wird und die erhaltenen Produkte summiert werden, eingeführt. Dieses Verfahren weicht also im wesentlichen nur insofern von den vorher beschriebenen qualitativen Charakterisierungsverfahren ab, als die zur qualitativen Wertung der mutagenen Wirkung des chemischen Stoffes geeigneten Daten willkürlich umgeformt und dadurch besser benutzbar gemacht werden, diese Umgestaltung hat jedoch keinerlei prinzipielle Grundlage, sie ist völlig formal. Es liegt auf der Hand, daß dieses vor allem auf qualitativen Daten beruhende Verfahren keine zuverlässigen quantitativen Werte zur Charakterisierung der mutagenen Wirkung zu liefern vermag.

Es wurden auch schon und werden zur Zeit Bakteriophagen zum Studium des Wirkungsmechanismus jeweils eines chemischen Stoffes oder einer Gruppe von chemischen Stoffen eingesetzt. Zum Nachweis wird die mutagene Wirkung des chemischen Stoffes genutzt, das heißt es wird untersucht, welche Veränderung an den Phagen durch den chemischen Stoff verursacht wird (Spezifitschnosti chimitscheskowo mutagenesa, Nauka, Moskau, *1968,* 76 bis 80 und 80 bis 84 ; Biochim. Biophys. Hung. *10* [1975], 115 bis 120 ; J. Virol. *19* [1976], 318 bis 324 und *24* [1977], 716 bis 719 ; Biochem. Biophys. Acta *546* [1979], 495 bis 506). Diese Arbeiten waren nicht darauf gerichtet und auch die Ergebnisse trugen nicht dazu bei, mit ihrer Hilfe Mutagenitätsprüfverfahren zu erarbeiten.

Im Fachschrifttum wird ein ausgesprochen auf der Phagenmutagenität beruhendes Prüfverfahren in Bezug auf geradzahlige T-Phagen (T2, T4) empfohlen. Von japanischen Verfassern (Ikeda, Iijima : J. Gen. Appl. Microbiol. *11* [1965], 129 bis 135) wurden T2-Phagen verwendet. In diesem Verfahren wird die spezifische Mutation (T2h) der wilden T2-Phage (T2h+) auf die Wirtszelle E. coli B/2 zur Untersuchung der mutagenen Aktivität der Chemikalien zunutze gemacht. In der Praxis fand dieses Verfahren in den letzten 15 Jahren keine Verbreitung. Das hat folgende prinzipielle beziehungsweise praktische Gründe :

a) Die Nucleinsäure der T2-Phage hat größenordnungsmäßig ein Molekulargewicht von $10^8$. Von dieser großen Nucleinsäure nimmt an der Mutation h+ ⟶ h offensichtlich nur ein sehr kleiner Teil teil, das heißt es beteiligen sich an ihr nur einige Gene. Es ist also wenig wahrscheinlich, daß vom T2-Phagengenom nur das beziehungsweise die die für den Prüfversuch ausgewählte Mutation verursachende(n) Gen(e) mit dem chemischen Stoff und nur sie in Wechselwirkung treten. Dieser Umstand senkt die Empfindlichkeit des Verfahrens stark.

b) Mit diesem Umstand hängt auch die Tatsache zusammen, daß die Wahrscheinlichkeit der letalen beziehungsweise tödlichen Mutation, das heißt der Phageninaktivierung, wesentlich höher als die Wahrscheinlichkeit der für den Prüfversuch verwendeten Mutation ist. So kann die auf die Wirkung des chemischen Stoffes zustandekommende Phagenvernichtung auch das Entstehen der ausgewählten Mutante verdecken. Diese Erscheinung kann bei besonders hohen mutagenen Stoffkonzentrationen auftreten.

c) Bei der praktischen Durchführung des genannten bekannten Verfahrens besteht ein Nachteil auch darin, daß der mutagene chemische Stoff direkt dem Komplex von Phagen und Bakterien zugesetzt wird, weshalb seine bakterizide Wirkung zur Geltung kommen und das Wachsen der Wirtsbakterien verhindert werden kann, was eventuell auch das Auswerten vereiteln kann.

So sind in Mutation Research *65* (1979), 133 bis 226 auf Seite 148, vorletzte Zeile bis Seite 149, Zeile 8 3 Aussiebversuche beschrieben :

(1) Wirtsvermittelte Versuche. Auf Grund ihrer geringen Größe, welche eine Ansammlung in Kapilaren verhindert, wurden Phagen zur Verwendung in wirtsvermittelten Versuchen vorgeschlagen.

(2) Die Mutationsinduktion bei der Umwandlung von DNA wurde zur Veranschaulichung der Mutagenität N-Azetoxy-2-AAF verwendet.

(3) Mehrere direkt wirkende polycyclische aromatische Kohlenwasserstoffkarzinogene wurden in einem Versuch mit infektiöser Nukleinsäure E. coli Sphäroplast untersucht. Sphäroplaste und QB RNA oder ØX174 DNA der auf Indikatorbakterien aufgebrachten zu untersuchenden Chemikalie ausgesetzt und es wurde die Änderung im Plättchenbildungsvermögen der infizierten Sphäroplaste bestimmt.

Beim Verfahren (1) dieser Druckschrift sind jedoch bei der Durchführung der Untersuchungen die Bedingungen nicht *in vitro*. In den Verfahren (2) und (3) dienen nicht Phagen im ganzen, sondern bloße Nukleinsäuren, das heißt transformierende Nukleinsäure beziehungsweise Phagen-Nukleinsäure als Untersuchungsstoffe. Außerdem sind die Nukleinsäuren von Phagen QB beziehungsweise ØX174 einsträngige RNS beziehungsweise DNS.

Das auf der λ-Phageninduktion beruhende im Fachschrifttum Indukttest genannten Verfahren bezieht sich auch auf Phagen (J. Gen. Appl. Microbiol. *11* [1965], 129 bis 135 ; Proc. Natl. Acad. Sci. *73* [1976], 3 700 bis 3 704, zusammengefaßt in Mutation Research, *65* [1979], 133 bis 226 auf Seite 148, Zeilen 7 bis 36). Dieses Verfahren unterscheidet sich jedoch schon prinzipiell von den vorherigen darin, daß der mutagene chemische Stoff mit dem ganzen (lysogenisierten) Bakteriumchromosom in Wechselwirkung tritt. Die vom mutagenen chemischen Stoff hervorgerufene DNS-Schädigung führt zu einer Stoffwechseländerung, die wiederum die Induktion der λ-Prophage verursacht. Im Indukttest ist das Erscheinen von Phagen also nur ein Indikator des Auftretens der Mutation, die mutagene Wirkung ist jedoch nicht direkt auf die Phagen gerichtet. Das mit dem Grundverfahren zusammenhängende Problem besteht darin, daß das Verfahren wenig empfindlich ist, das heißt, daß eine hohe mutagene Konzentration des chemischen Stoffes zum Auslösen der Induktion notwendig ist (J. Gen. Appl. Microbiol. *11* [1965], 129 bis 135). Wie in jedem bekannten Verfahren zur Untersuchung einer nicht-letalen Mutation kann der zu untersuchende chemische Stoff auch in diesem Fall neben der Prophageninduktion schädliche Wirkungen ausüben. Diese Wirkung kann einerseits direkt auf die Phage, andererseits auf die lysogenisierte Wirtsbakterie gerichtet sein. Im ersten Fall wird die Phage inaktiviert, während sich im zweiten Fall die Phagenerzeugungsfähigkeit der Zelle verringert. Beide erwähnten Wirkungen sind vorwiegend bei hohen mutagenen Konzentrationen des chemischen Stoffes zu erwarten und können die Prophageninduktion verdecken. Dadurch kann der Prüfversuch ein falsch negatives Ergebnis liefern.

Der gemeinsame Mangel der angegebenen Verfahren besteht darin, daß für die quantitative Wertung der mutagenen Wirkung nur eine sehr primitive und willkürliche Verfahrensweise gewählt wird. Auf Grund der Charakterisierung kann nur eine positive (leicht oder stark) beziehungsweise negative Entscheidung getroffen werden. Eine solche im wesentlichen aus 2 Stufen bestehende Skala kann überhaupt nicht die Erfahrung widerspiegeln, daß die Stärkeskala der mutagenen/karzinogenen Wirkung der chemischen Stoffe 6 bis 7 Größenordnungen umfaßt (J. Toxicol. Environm. Health *6* [1980], 1 133 bis 1 177).

Der Erfindung liegt die Aufgabe zugrunde, unter Behebung der Nachteile der bekannten Verfahren ein Verfahren zur Bestimmung der mutagenen Wirkung von chemischen Stoffen, welches zuverlässig und empfindlich ist, einfach, mit geringem Aufwand und schnell sowie mit gut zu erhaltenden und zu handhabenden Untersuchungsstoffen durchgeführt werden kann sowie eine quantitative Aussage über die mutagene Wirkung und ihre Kinetik bei guter Auswertbarkeit gibt, zu schaffen.

Das Obige wurde überraschenderweise durch die Erfindung erreicht.

Es wurde nämlich überraschenderweise festgestellt, daß bestimmte Phagen als Untersuchungsstoffe zur quantitativen Bestimmung der mutagenen Wirkung von chemischen Stoffen bei Erreichung des Obigen eingesetzt werden können. Solche sind Phagen, die eine kein lysogenes Gen enthaltende doppelsträngige DNS haben. In diesen Phagen führt nach eigenen Untersuchungen die Dimerisation einer einzigen Pyrimidinbase schon zum Zugrundegehen der ganzen Phase. In den Bakterien müssen dagegen demgegenüber zur Auslösung der Reversion der Mutante eventuell mehrere oder umfangreichere, genau nicht bekannte chemische Veränderungen innerhalb eines einzigen Objektes erfolgen. Demzufolge können die teilweise veränderten Bakterien bei der Auswertung der Untersuchung als intakt erscheinen. Die Phagen zeigen also im Gegensatz zu den Bakterien schon die erste Schädigung zuverlässig an. Diese Phagen üben ähnlich wie die übrigen Phagen in Abwesenheit von Wirtszellen keine Lebensfunktionen aus, weswegen auch nicht mit der Gefahr gerechnet werden muß, daß sich das Untersuchungsobjekt nach Ablauf der schädlichen chemischen Reaktion durch biologische Reaktionen regeneriert und sich bei der endgültigen Auswertung als heil zeigt und dadurch ein falsches Ergebnis angibt. Eine weitere vorteilhafte Eigenschaft der Phagen besteht darin, daß sie in Pufferlösungen (pH-Wert von 6,0 bis 8,0, wie etwa 7,0) im Kühlschrank ohne besondere Behandlung, zum Beispiel Lüften, Zusatz von Nährböden oder Umimpfen, praktisch unbegrenzt gelagert werden können, weswegen ihr Erhalten und ihre Nutzung wesentlich einfacher ist und mit einem wesentlich geringeren Aufwand verbunden ist als die von Mikroorganismen.

Frühere eigene Untersuchungen (Studia Biophys. *33* [1972], 121 waren nur mit der durch ultraviolettes Licht verursachten Inaktivierung der T7-Phagen befaßt. Im Laufe der nunmehrigen Versuche wurde der zeitliche Ablauf der auf die Wirkung von chemischen Stoffen hin erfolgenden Inaktivierung der obigen Phagen untersucht und festgestellt, daß die Zeit, in welcher ein gegebener chemischer Stoff in der untersuchten Konzentration die gleiche Phagenvernichtung verursacht wie das ultraviolette Licht, das in der Phagenbevölkerung durchschnittlich 1 Pyrimidindimer hervorruft, angegeben werden kann. Der Erfindung liegt die Feststellung zugrunde, daß das Produkt der Konzentration des chemischen Stoffes und der Zeit der die erwähnte Phagenvernichtung verursachenden Wirkung eine Konstante ist und die mutagene Wirkung des gegebenen chemischen Stoffes quantitativ charakterisiert.

Gegenstand der Erfindung ist daher ein Verfahren zur quantitativen Bestimmung der mutagenen Wirkung von chemischen Stoffen auf Phagen durch eine *in vitro* durchgeführte Untersuchung, welches dadurch gekennzeichnet ist, daß zur statistischen Auswertung der Phagen einer mit einer Pufferlösung mit einem pH-Wert von 6,0 bis 8,0 bereiteten Suspension der Phagen von einer Konzentration von $10^6$ bis $10^8$ Phagen/ml eine bekannte Menge eines in Wasser, in einer höchstens 20 vol.-%-igen wäßrigen Methanol- oder Äthanollösung oder in einer höchstens 38 vol.-%-igen wäßrigen Dimethylsulfoxydlösung löslichen oder sich mit diesen Lösungsmitteln vermischenden zu untersuchenden chemischen Stoffes zugesetzt wird, wobei als Phagen solche, welche eine kein lysogenes Gen enthaltende doppelsträngige DNS aufweisen, eingesetzt werden, dann der Suspension nach der Zugabe des chemischen Stoffes sofort eine Probe entnommen wird und die Ausgangsphagenkonzentration der Probe [N(O)] durch in an sich bekannter Weise auf einer entsprechenden Wirtszelle durchgeführte Züchtung beziehungsweise Kultivierung bestimmt wird und die den chemischen Stoff enthaltende Phagensuspension auf einer Temperatur von 20 bis 37,5 °C, insbesondere 37 °C, gehalten wird, der Suspension von Zeit zu Zeit eine Probe entnommen wird und die Konzentration der überlebenden Phagen durch in an sich bekannter Weise auf einer entsprechenden Wirtszelle durchgeführte Züchtung beziehungsweise Kultivierung zum Zeitpunkt der Probenahme [N(t)] bestimmt wird sowie auf Grund der erhaltenen Angaben der für die mutagene Wirkung charakteristische Zahlenwert errechnet wird.

Das Berechnen des für die mutagene Wirkung charakteristischen konstanten Zahlenwertes kann wie folgt vorgenommen werden. Es werden die zu den einzelnen Zeitpunkten gehörenden Quotienten ln [N(t)/N(O)] berechnet, durch Berechnung oder auf graphischem Wege die dem Wert ln [N(t)/N(O)] $= - 1$ zugehörige Zeit bestimmt und dieser Wert mit der Konzentration des zu untersuchenden chemischen Stoffes multipliziert.

Der als Endergebnis der beschriebenen Bestimmung und Berechnung erhaltene Wert wird im folgenden « pyrimidindimeräquivalente Dosis » genannt. Die pyrimidindimeräquivalente Dosis ist für jede einzelne Verbindung ein konstanter Wert und charakterisiert die mutagene Wirkung der Verbindung quantitativ. Die pyrimidindimeräquivalent Dosis und die mutagene Wirkung ändern sich in einander entgegengesetztem Sinne, das heißt, daß je höher die pyrimidindimeräquivalente Dosis einer gegebenen Verbindung ist, um so geringer die mutagene Wirkung der Verbindung ist.

Zur Veranschaulichung ist es in der Praxis zweckmäßig, zur Charakterisierung der mutagenen Wirkung den Kehrwert dieses Wertes, der sich in gleichem Sinne wie die mutagene Wirkung ändert, statt der pyrimidindimeräquivalenten Dosis zu verwenden. Wenn die reziproke Skala zur Charakterisierung der mutagenen Wirkung verwendet wird und die Zeit bei der Berechnung in Minuten und die Konzentration des zu untersuchenden chemischen Stoffes in Mol/l angegeben werden, dann haben die nicht beziehungsweise weniger gefährlichen Stoffe Werte von 0 bis 1 und die stark mutagenen Stoffe Werte über 1.

Im Hinblick darauf, daß für die zur Inaktivierung der Phagen führenden chemischen Reaktionen die Gesetze der großen Zahlen gültig sind, kann die pyrimidindimeräquivalente Dosis mit zuverlässiger Genauigkeit nur dann bestimmt werden, wenn sehr viele Untersuchungsobjekte (Phagen) in den Versuch einbezogen werden. Daher müssen die Phagen in ausreichend hoher Konzentration eingesetzt werden, wie es oben festgelegt ist. Vorzugsweise wird die Phagenkonzentration zu $8 \times 10^6$ bis $10^8$ Phagen/ml, insbesondere $8 \times 10^6$ bis $5 \times 10^7$ Phagen/ml, ganz besonders $10^7$ bis $5 \times 10^7$ Phagen/ml, beispielsweise $10^7$ Phagen/ml, gewählt.

Vorzugsweise werden als Phagen die T7-Phagen eingesetzt.

Da die Phagen am vorteilhaftesten in Wasser erhalten werden können, ist das erfindungsgemäße Verfahren besonders vorteilhaft zur Durchführung der Bestimmung der mutagenen Wirkung von wasserlöslichen oder sich mit Wasser vermischenden chemischen Stoffen anwendbar.

Unter Berücksichtigung der pH-Empfindlichkeit der Phagen können die Bestimmungen mit solchen chemischen Stoffen, welche den pH-Wert der Puffer unter 6,0 senken, nicht durchgeführt werden.

Es ist bevorzugt, als Phagensuspension eine solche, welche mit einer Pufferlösung mit einem pH-Wert von 7,0 bereitet worden ist, einzusetzen.

Gegenüber dem Verfahren der deutschen Offenlegungsschrift 30 18 900 bringt das erfindungsgemäße Verfahren den großen Vorteil mit sich, daß zur Auswertung der letalen Mutation von Bakteriophagen eine kürzere Zeit erforderlich ist, also das erfindungsgemäße Bestimmungsverfahren viel schneller ist. Das erfindungsgemäße Verfahren hat den weiteren Vorteil, daß bei ihm die Statistik sich aus einer höheren Anzahl, nämlich der hohen Anzahl von $10^6$ bis $10^8$ Versuchsobjekten (Bakteriophagen) durchführen läßt. Ein weiterer Vorteil des erfindungsgemäßen Verfahrens gegenüber dem genannten bekannten Verfahren ist, daß das erstere einen Zahlenwert, welcher für die mutagene Aktivität der untersuchten Chemikalie charakteristisch ist, angibt. (Dieser Wert ist der Wahrscheinlichkeit, mit welcher eine Mutation durch die Konzentrationseinheit der Chemikalie verursacht wird, umgekehrt proportional). Er ist also von der Konzentration der Chemikalie unabhängig und ermöglicht einen Vergleich der mutagenen Wirksamkeit der chemischen Stoffe. Darüber hinaus ist das erfindungsgemäße Verfahren zur vollautomatisierten Durchführung der Untersuchung geeignet.

Im Gegensatz zum Verfahren (1) in Mutation Research, Band 65 (1979), Seiten 133 bis 226 auf Seite 148, vorletzte Zeile bis Seite 149, Zeile 1 handelt es sich beim erfindungsgemäßen Verfahren um eine Untersuchung *in vitro*, was für es charakteristisch ist.

Im Gegensatz zu den Verfahren (2) und (3) in Mutation Research, Band 65 (1979), Seiten 133 bis 226 auf Seite 149, Zeilen 1 bis 8 werden beim erfindungsgemäßen Verfahren doppelsträngige DNS enthaltende Phagen als Untersuchungsstoffe verwendet, wie es bereits weiter oben festgelegt wurde, das heißt, daß die Verwendung der bloßen Nukleinsäuren ausgeschlossen ist. Die im erfindungsgemäßen Verfahren verwendeten doppelsträngigen DNS enthaltenden Phagen als Untersuchungsstoffe haben gegenüber den in der genannten Druckschrift verwendeten bloßen Nukleinsäuren den Vorteil, daß ihre Struktur, in welcher sich die doppelsträngige DNS in Wechselwirkung mit Proteinen befindet, der Struktur der Chromosomen von höheren Organismen nähersteht. Die Reaktionen mit den zu untersuchenden chemischen Stoffen laufen im Falle von ganzen Phagen den Chromosomen ähnlicher ab als im Falle von bloßen Nukleinsäuren.

Die Erfindung wird an Hand der folgenden Beispiele näher erläutert.

Beispiel 1

Bestimmung der mutagenen Wirkung von Vitamin $B_1$

Es wurde zu 2 ml einer mit einem Phosphatpuffer aus 0,13 g Magnesiumsulfat ($MgSO_4$), 1 g Ammoniumchlorid ($NH_4Cl$), 3 g Kaliumdihydrogenphosphat ($KH_2PO_4$) und 6 g Dinatriumhydrogenphosphat ($Na_2HPO_4$) in 1 l Wasser mit einem pH-Wert von 7,0 bereiteten T7-Phagensuspension mit einer Konzentration von $1 \times 10^7$ bis $5 \times 10^7$ Phagen/ml die identische Menge von im auf 37 °C erwärmten Phosphatpuffer derselben Zusammensetzung in einer Konzentration von 0,2 Mol/l (27,8 g/l) gelöstem Vitamin $B_1$ zugegeben. Der Suspension wurde sofort eine Probe entnommen und nach der unten beschriebenen Verfahrensweise wurde die Ausgangsphagenkonzentration [N(O)] bestimmt. Die Suspension wurde in einen auf 37 °C geheizten Thermostaten eingebracht und zur Bestimmung der Konzentration [N(t)] der die Wirkung von Vitamin $B_1$ nach dem Ablauf der jeweiligen Zeit (t) überlebenden Phagen wurde der Suspension 45, 90, 135, 180, 300 und 360 Minuten nach dem Beginn der Bebrütung beziehungsweise Inkubation je 1 Probe mit einem Volumen von 0,05 ml entnommen. Als Blindversuchs- beziehungsweise Kontrollprobe wurde eine T7-Phagensuspension, welche abgesehen vom Zusatz von Vitamin $B_1$ in allem gleich behandelt wurde, verwendet. Der Blindversuchs- beziehungsweise Kontrollsuspension wurde zu gleichen Zeitpunkten wie der mit Vitamin $B_1$ bebrüteten beziehungsweise inkubierten Suspension eine Probe entnommen. Nach insgesamt 6 Probenahmen wurde der Versuch abgebrochen.

Die Phagenkonzentration wurde mittels der von der Anmelderin geänderten (Karczag : Dissertation, 1976 ; Karczag, Fidy, Aczél : Studia Biophys. *30* [1972] ; Rontó : Disseration, 1979) Titration nach Gratia (Adams : Bacteriophages, Intersci. New York [1959]) wie folgt bestimmt : Die entnommenen Proben wurden im ersten Schritt mit dem Phosphatpuffer der oben angegebenen Zusammensetzung auf 5 ml und dann der ersten Verdünnung entnommene 0,05 ml Proben jeweils erneut auf 5 ml verdünnt. Der zweiten Verdünnung entnommene 0,1 ml Proben wurden mit 1,5 ml dünnem Agar und 0,5 ml E. coli B/r Wirtszellensuspension (Suspendiermedium : Nährbouillon, Konzentration : $10^8$/ml) verrührt und das Gemisch wurde in eine Petri-Schale gefüllt. Die Verdünnung war für die biologische Titration geeignet, wenn in je 1 Petri-Schale 50 bis 300 aktive Phagen gelangten. Die biologische Titration wurde zweckmäßig 4- bis 5-mal wiederholt. Nach dem Erstarren des Oberflächenagars wurden die Petri-Schalen 2 bis 3 Stunden lang in einem Thermostaten bei 37 °C bebrütet beziehungsweise inkubiert. Nach dem Ende der Bebrütung beziehungsweise Inkubation zeigte je 1 Plaque die Stelle der keine letalen Mutation erleidenden Phagen in den einzelnen Petri-Schalen an ; die Zahl der Leerflecke gibt also bei der Probenahme die Zahl der in die Petri-Schale gelangten aktiven Phagen an.

Es wurde der Durchschnitt der bei den von gleichen Probenahmen durchgeführten Titrationen erhaltenen Plaques gebildet und aus den Verdünnungsverhältnissen wurde berechnet, wie viele heile Phagen die entnommene Probe ursprünglich enthielt. Aus der ins Verhältnis zum Volumen der Suspension gesetzten Menge der Probe wurde die Menge der überlebenden Phagen [N(t)] bestimmt.

Für den Zeitpunkt jeder einzelnen Probeentnahme wurde der Bruch 1n [N(t)/N(O)] errechnet und dieser Wert in Abhängigkeit von der Zeit graphisch (zweckmäßig in einem halblogarithmischen Koordinatensystem) dargestellt. Der Anfangsabschnitt der Kurve der so erhaltenen Dosiswirkung wurde durch eine Gerade angenähert und so graphisch der Wert der 1n [N(t)/N(O)] = —1 zugehörigen Bebrütungs- beziehungsweise Inkubationszeit (in Minuten ausgedrückt) bestimmt. Durch die Multiplikation dieser Bebrütungs- beziehungsweise Inkubationszeit (291 Minuten) mit der in Mol/l ausgedrückten Konzentration von Vitamin $B_1$ (0,1 Mol/l) wurde die pyrimidindimeräquivalente Dosis, deren Wert 29 (Mol · Minute/l) betrug, erhalten. Der Kehrwert der pyrimidindimeräquivalenten Dosis betrug 0,03 (l/Mol · Minute).

Beispiel 2

Nach der im Beispiel 1 beschriebenen Verfahrensweise wurden auch die pyrimidindimeräquivalenten Dosen beziehungsweise deren Kehrwerte von anderen Verbindungen, welchletztere in der folgenden Tabelle zusammengestellt sind, bestimmt.

Tabelle

| Untersuchte Verbindung | Kehrwert der pyrimidin-dimeräquivalenten Dosis in $\dfrac{1}{\text{Mol . Minute}}$ |
|---|---|
| Nitrosomethylharnstoff | 0,76 |
| Äthylenimin | 6,7 |
| 4-Desoxy-4-amino-10-methylfolsäure {4-Amino-N$_{10}$-methyl-pteroyl-glu-taminsäure} [Methotrexat] | 11 |
| N-(Acetyl)-äthylenimin | 33 |
| {2-(Chlor)-äthyl}-{2-(hydroxy)--äthyl}-sulfid [monofunktioneller Schwefelsenf] | 38 |
| 1,4-Di-{2'-(methylsulfonyl)-oxy-äthylamino}-1,4-di-{desoxy}-ery-thrit-dimethylsulfat [Lycurim] | 111 |
| Di-{2-(chlor)-äthyl}-sulfid [bifunktioneller Schwefelsenf] | 1 000 |

Die in der obigen Tabelle zusammengestellten Daten stimmen mit der Erfahrung, daß die schädliche Wirkung der untersuchten chemischen Stoffe tatsächlich in der angegebenen Reihenfolge zunimmt, völlig überein und das Verhältnis ihrer in Tierversuchen bestimmten letalen Dosen entspricht den aus den obigen Werten berechenbaren Quotienten.

Nach den Untersuchungen betrug die Fehlergrenze bei den Versuchen weniger als 10 %.

**Patentansprüche**

1. Verfahren zur quantitativen Bestimmung der mutagenen Wirkung von chemischen Stoffen auf Phagen durch eine *in vitro* durchgeführte Untersuchung, dadurch gekennzeichnet, daß man zur statistischen Auswertung der Phagen einer mit einer Pufferlösung mit einem pH-Wert von 6,0 bis 8,0 bereiteten Suspension der Phagen von einer Konzentration von $10^6$ bis $10^8$ Phagen/ml eine bekannte Menge eines in Wasser, in einer höchstens 20 vol.-%-igen wäßrigen Methanol- oder Äthanollösung oder in einer höchstens 38 vol.-%-igen wäßrigen Dimethylsulfoxydlösung löslichen oder sich mit diesen Lösungsmitteln vermischenden zu untersuchenden chemischen Stoffes zusetzt wobei man als Phagen solche, welche eine kein lysogenes Gen enthaltende doppelsträngige DNS aufweisen, einsetzt, dann der Suspension nach der Zugabe des chemischen Stoffes sofort eine Probe entnimmt und die Ausgangsphagenkonzentration der Probe durch in an sich bekannter Weise auf einer entsprechenden Wirtszelle durchgeführte Züchtung beziehungsweise Kultivierung bestimmt und die den chemischen Stoff enthaltende Phagensuspension auf einer Temperatur von 20 bis 37,5 °C hält, der Suspension von Zeit zu Zeit eine Probe entnimmt und die Konzentration der überlebenden Phagen durch in an sich bekannter Weise auf einer entsprechenden Wirtszelle durchgeführte Züchtung beziehungsweise Kultivierung zum Zeitpunkt der Probenahme bestimmt sowie auf Grund der erhaltenen Angaben den für die mutagene Wirkung charakteristischen Zahlenwert errechnet.

2. Verfahren nach Anspruch 1, dadurch gekennzeichnet, daß man als Phagen die T7-Phagen einsetzt.

3. Verfahren nach Anspruch 1 oder 2, dadurch gekennzeichnet, daß man die Phagenkonzentration zu $8 \times 10^6$ bis $10^8$ Phagen/ml, insbesondere $8 \times 10^6$ bis $5 \times 10^7$ Phagen/ml, wählt.

4. Verfahren nach Anspruch 1 bis 3, dadurch gekennzeichnet, daß man als Phagensuspension eine solche, welche mit einer Pufferlösung mit einem pH-Wert von 7,0 bereitet worden ist, einsetzt.

## Claims

1. A process for the quantitative determination of the mutagenic effect of chemical substances on phages by a test carried out *in vitro*, characterized in that for the statistic evaluation of the phages to a suspension of the phages having a concentration of $10^6$ to $10^8$ phages/ml prepared with a buffer solution having a pH of 6,0 to 8,0 a known amount of a chemical substance to be tested and being soluble in or mixable with water, utmost 20 %, by volume, aqueous methanol or ethanol solution or utmost 38 %, by volume, aqueous dimethyl sulfoxid solution is added, as phages those are being used which comprise no lysogenic gene containing doublestranded DNS, then after the addition of the chemical substance immediately a sample is taken from the suspension and the starting phages concentration of the sample is determined by breeding and cultivation, respectively in a manner known *per se* on a corresponding host cell, and the phages suspension containing the chemical substance is maintained at a temperature of 20 to 37,5 °C, a sample is taken from the suspension from time to time and the concentration of the surviving phages is determined at the point of sampling by breeding and cultivation, respectively in a manner known *per se* on a corresponding host cell, and on the basis of the obtained data the numeric value being characteristic for the mutagenic effect is calculated.

2. A process according to claim 1, characterized in that as phages the T7-phages are used.

3. A process according to claim 1 or 2, characterized in that a phages concentration of $8 \times 10^6$ to $10^8$ phages/ml, especially $8 \times 10^6$ to $5 \times 10^7$ phages/ml, is used.

4. A process according to claim 1 to 3, characterised in that as phages suspension a such one is used which has been prepared with a buffer solution having a pH of 7,0.

## Revendications

1. Procédé de détermination quantitative de l'effet mutagène de substances chimiques sur des phages par un essai effectué *in vitro*, caractérisé en ce que, pour évaluer statistiquement les phages, on ajoute à une suspension des phages préparée avec une solution-tampon d'une valeur de pH de 6,0 à 8,0, d'une concentration de $10^6$ à $10^8$ phages/ml, une quantité connue d'une substance chimique à examiner soluble dans l'eau, dans une solution aqueuse de méthanol ou d'éthanol à 20 % en volume au plus ou dans une solution aqueuse de diméthylsulfoxyde à 38 % en volume au plus, ou se mélangeant avec ces solvants, en utilisant, comme phages, des phages comportant un ADN (acide désoxyribonucléique) à double hélice ne contenant pas de gène lysogène, puis l'on prélève immédiatement de la suspension, après avoir ajouté la substance chimique, un échantillon et l'on détermine la concentration initiale en phages de l'échantillon par croissance ou culture effectuée de façon connue en soi sur une cellule-hôte correspondante, et l'on maintient la suspension de phages renfermant la substance chimique à une température de 20 à 37,5 °C, on prélève de temps en temps un échantillon de la suspension et l'on détermine la concentration en phages survivants par une croissance ou culture effectuée de façon connue en soi sur une cellule-hôte correspondante au moment du prélèvement de l'échantillon, et l'on calcule également, sur la base des indications obtenues, la valeur numérique caractéristique de l'effet mutagène.

2. Procédé selon la revendication 1, caractérisé en ce qu'on utilise, comme phages, les phages T7.

3. Procédé selon la revendication 1 ou 2, caractérisé en ce qu'on choisit la concentration en phages de $8 \times 10^6$ à $10^8$ phages/ml, notamment à $8 \times 10^6$ à $5 \times 10^7$ phages/ml.

4. Procédé selon l'une des revendications 1 à 3, caractérisé en ce qu'on utilise, comme suspension de phages, une suspension qui a été préparée avec une solution-tampon d'une valeur de pH de 7,0.